(19) Europäisches Patentamt
European Patent Office
Office européen des brevets

(11) **EP 0 965 346 B1**

(12) **EUROPEAN PATENT SPECIFICATION**

(45) Date of publication and mention of the grant of the patent:
**23.07.2003 Bulletin 2003/30**

(51) Int Cl.7: **A61K 31/715**, A61P 11/00

(21) Application number: **99200519.9**

(22) Date of filing: **05.11.1991**

(54) **Use of acetylated mannan derivatives for treating chronic respiratory diseases**

Verwendung von acetylierten Mannanderivaten zur Behandlung von chronischen Atemwegserkrankungen

Utilisation des derivés acetylés du mannane pour le traitement des maladies respiratoires chroniques

(84) Designated Contracting States:
**DE FR GB IT**

(43) Date of publication of application:
**22.12.1999 Bulletin 1999/51**

(62) Document number(s) of the earlier application(s) in accordance with Art. 76 EPC:
**92900586.6 / 0 611 304**

(73) Proprietor: **CARRINGTON LABORATORIES, INC.**
**Irving, TX 75038 (US)**

(72) Inventors:
- **McAnalley, Bill H.**
  **Grand Prairie, Texas 75052 (US)**
- **Carpenter, Robert H.**
  **Bastrop, Texas 78602 (US)**
- **McDaniel, Harley R.**
  **Grand Prairie, Texas 75053 (US)**

(74) Representative: **Fisher, Adrian John**
**CARPMAELS & RANSFORD**
**43 Bloomsbury Square**
**London WC1A 2RA (GB)**

(56) References cited:
**US-A- 3 920 816**

- **D. WOMBLE ET AL.: "Enhancement of allo-responsiveness of human lymphocytes by acemannan." INT. J. IMMUNOPHARMACOL., vol. 10, no. 8, 1988, pages 967-974, XP002118805**
- **J. MEASEL ET AL: "The effect of carrisyn on the immune system." FED. AM. SOC. EXP. BIOL. J., vol. 2, no. 4, 1988, XP002118806**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

### A. Field of the Invention

The invention pertains to uses of biological response modifying agents. More particularly, this invention relates to the therapeutic use of a polysaccharide substance which is an acetylated mannan in the manufacture of a medicament for reducing symptoms associated with chromic respiratory diseases.

### B. Description of the General Background Information

Aloe is a member of the lily family. Aloe vera contains two major liquid sources, a yellow latex (exudate) and the clear gel (mucilage). The dried exudate of Aloe barbadensis Miller leaves is referred to as aloe. The commercial name is Curacao aloe. It is composed mainly of aloin, aloe-emodin and phenols. Bruce, South African Medical Journal, 41:984 (1967); Morrow et al., Archives of Dermatology, 116:1064 -1065 (1980); Mapp et al., Planta Medica, 18:361-365 (1970); Rauwald, Archives Pharmazie, 315:477-478 (1982). A number of phenolics, including anthraquinones and their glycosides, are known to be pharmaceutically active. Bruce, Excelsa, 5:57-68 (1975); Suga et al., Cosmetics and Toiletries, 98:105-108 (1983).

The mucilaginous jelly from the parenchymal cells of the plant is referred to as Aloe vera gel. There are generally no anthraquinones to decompose and cause discoloration of the gel unless the gel is contaminated by an improper processing technique. Aloe vera gel is about 98.5% water by weight. More than 60% of the total solid is made up of polysaccharides of carbohydrate origin. Organic acids and inorganic compounds, especially calcium oxalate, account for the remainder of the solid.

Aloe vera was the traditional medicine of many cultures as an anthelmintic, cathartic and stomachic and was used inter alia for leprosy, burns and allergic conditions. Cole et al., Archives of Dermatology and Syphilology, 47:250 (1943); Chopra et al., Glossary of Indian Medicinal Plants, Council of Scientific and Industrial Research, New Delhi (1956); Ship, Journal of the American Medical Association, 238(16):1770-1772 (1977); Morton, Atlas of Medicinal Plants of Middle American Bahamas to Yucatan, Charles C. Thomas Publisher, 78-80 (1981); Diez-Martinez, La Zabila, Communicado NO. 46 Sobre Recursos Bioticos Potenciales del Pais, INIREB, Mexico (1981); Dastur, Medicinal Plants of India and Pakistan: D.B. Taraporevala Sons & Co., Private Ltd., Bombay 16-17 (1962).

Aloe vera has been featured extensively in the field of dermatology, especially for treating radiation-caused skin conditions. Mackee, X-rays and Radium in the Treatment of Diseases of the Skin, 3rd Ed., Lea and Febiger, Philadelphia, 319-320 (1938); Rovatti et al., Industrial Medicine and Surgery, 28:364-368 (1959); Zawahry et al., Quotations From Medical Journals on Aloe Research, Ed. Max B. Skousen, Aloe Vera Research Institute, Cypress, California, 18-23 (1977); Cera et al., Journal of the American Animal Hospital Association, 18:633-638 (1982). The body of scientific literature documenting medical applications in digestive problems, as a virucidal, bactericidal and fungicidal agent and in gynecological conditions is extensive and has been adequately reviewed by Grindley et al., [Journal of Ethnopharmacology, 16:117-151 (1986)].

Depending on the way the leaves are processed, mucilage and sugars are the major components of the dehydrated gel. The sugars found are galactose, glucose, mannose, rhamnose, xylose and uronic acids. Although reports conflict, the mucilage is mainly composed of mannan or glucomannan. Eberendu et al., The Chemical Characterization of Carrisyn® (in preparation); Mandal et al., Carbohydrate Research, 86:247-257 (1980b); Roboz et al., Journal of the American Chemical Society, 70:3248-3249 (1948); Gowda et al., Carbohydrate Research, 72:201-205 (1979); Segal et al., Lloydia, 31:423 (1968).

Prior to this work, the controversy over the identity of the active substance(s) in Aloe vera had not been settled. It is therefore important to clearly distinguish between the components present in the gel and those found in the exudates. A majority of the gel is a mucilage of mainly polysaccharide nature with minor amounts of various other compounds. It has been observed that in some of the activities there may be some synergistic action between the polysaccharide base and other components. Leung, Excelsa 8:65-68 (1978); Henry, Cosmetics and Toiletries, 94:42-43, 46, 48, 50 (1979). For example, several workers report that the effective components for wound healing may be tannic acid [Freytag, Pharmazie, 9:705 (1954)] and a kind of polysaccharide. Kameyama, Wound-healing compositions from Aloe arborescens extracts. Japanese Patent#7856995, (1979).

However, there are many examples in the literature indicating that polysaccharides can exhibit pharmacological. and physiological activities without help from other components. Gialdroni-Grassi, International Archives of Allergy and Applied Immunology, 76(Suppl. 1):119-127 (1985); Ohno et al., Chemical and Pharmaceutical Bulletin, 33(6): 2564-2568 (1985); Leibovici et al., Chemico-Biological Interactions, 60:191-200 (1986); Ukai et al., Chemical and Pharmaceutical Bulletin, 31:741-744 (1983); Leibovici et al., Anticancer Research, 5:553-558 (1985). One such example

relates to development of atherosclerosis. Hyperlipidemia in the general population and especially in familial hypercholesterolemia is associated with coronary heart disease and death. In countries where dietary fiber intake is high, atherosclerosis appears to be uncommon. Trowell et al., Editors, Refined Carbohydrate Foods and Disease, London, Academic Press, 207 (1975). Pectin and guar are reported to lower cholesterol in normal and hyperlipidemic patients. Kay et al., American Journal of Clinical Nutrition, 30:171-175 (1977). Locust bean gum, a polysaccharide composed of mannose and galactose, decreased the plasma lipoprotein cholesterol concentrations in both normal and familial hypercholesterolemic subjects. Zavoral et al., American Journal of Clinical Nutrition, 38:285-294 (1983). Addition of guar gum to carbohydrate meals decreased the postprandial rise of glucose in both normal and diabetic subjects. Jenkins et al., Lancet, 2:779-780 (1977). Kuhl et al., in Diabetes Care, 6(2):152-154 (1983) demonstrated that guar gum exhibited glycemic control of pregnant insulin-dependent diabetic patients.

The antitumor activity of polysaccharides has been widely reported. Polysaccharides prepared from Lentinus cyathiformis are known to increase host defense against tumors. Rethy et al., Annales Immunologiae Hungaricae, 21: 285-290 (1981). There are several reports that polysaccharides from mushroom, yeast or bacterial extracts can elicit a high degree of host defense activity against viral and tumor infestations. Chihara, Nature, 222:687 (1969); Shwartzman et al., Proceedings of the Society for Experimental Biology and Medicine, 29:737-741 (1932); Suzuki et al., Journal of Pharmacobio-Dynamics, 7(7):492-500 (1984), also reported antitumor activity of a polysaccharide fraction (GF-1) extracted from cultured fruiting bodies of a fungus, Grifola frondosa. This fraction showed equivalent, high levels of inhibiting activity when administered intraperitoneally (IP), intravenously (IV) and intratumorally (IT). However, oral administration (PO) was not effective. The GF-1 fraction also exhibited antitumor action against the solid form of Meth A fibrosarcoma and MM 46 carcinoma in mice. Lentinan, which is a 6-branched β-1-3-linked glucan similar to GF-1, was ineffective against Meth A fibrosarcoma. Chihara, "The antitumor polysaccharide Lentinan: an overview;" Manipulation of Host Defense Mechanisms; Ed. by Aoki et al., Excerpta Medica, North Holland, 1-16 (1981); Sasaki et al., Carbohydrate Research, 47(1):99-104 (1976). Synthesized branched polysaccharides were reported to demonstrate activities against tumors. Matsuzaki et al., Makromol. Chem., 186(3):449-456 (1985). Matsuzaki et al. [Makromol., Chem., 187(2):325-331 (1986)] synthesized branched polysaccharides, which showed significant activities, from ivory nut mannan (β-(1-4)-D-mannopyranose) and β-(1-4)-linked glucomannan. A partially acetylated linear β-(1-3)-D-mannan extracted from fruit bodies of Dictyophoria indusiata Fisch, also exhibited antitumor activity. Hara, Carbohydrate Research, 143:111 (1982). It appears that antitumor action depends on the type of polymer main chain and its degree of polymerization, because B-(1-3)-glucan-type polymers show higher antitumor activity than β-(1-4)-glucan and hemicellulosic polymers. Matsuzaki et al., Makromol. Chem., 187:325-331 (1986). A carboxymethylated derivative of β-(1-3)-glucan obtained from bacterial culture filtrate caused severe cell loss from established sarcoma 180 tumors within 2 hours after the injection of the derivative. Baba, Journal of Immunopharmacology, 8(6):569-572 (1986). The same author observed a compensatory increase in polymorphonuclear leukocytes due to injection of the substance. Incidentally, bestatin, a dipeptide known to possess immune-modulating and antitumor activity [Ishizuka, Journal of Antibiotics, 32:642-652 (1980)], influenced neither the tumor yield nor the polymorphonuclear leukocyte count. Baba et al., supra.

There are numerous reports on the antitumor effect of sulfated polysaccharides, including heparin [Jolles et al., Acta Univ. Int. Cancer, 16:682-685 (1960); Suemasu et al., Gann. 61(2):125-130 (1970)], sulfated laminaran and dextran [Jolles et al., British Journal of Cancer, 17:109-115 (1963)]. Yamamoto et al., in Japanese Journal of Experimental Medicine, 54:143-151 (1984), reported enhancement of antitumor activity of a fucoidan fraction by further sulfation. The sulfated product demonstrated activity against L-1210 leukemia. Polysaccharides with sulfate groups are also reported to be human T cell mitogens and murine polyclonal B cell activators. Sugawara et al., Microbiological Immunology, 28(7):831-839 (1984). Generally, homo-polysaccharides of high molecular weight with sulfate groups possess these properties. Dorries, European Journal of Immunology, 4:230-233 (1974); Sugawara et al., Cell Immunology, 74:162-171 (1982).

It has been reported that glucan extracted from the yeast Saccharomyces cervisiae is a modulator of cellular and humoral immunity. Wooles et al., Science, 142:1078-1080 (1963). The polysaccharide also stimulated proliferation of murine pluripotent hematopoietic stem cells, granulocyte macrophage colony-forming cells and cells forming myeloid and erythroid colonies. Pospisil et al., Experientia, 38:1232-1234 (1982); Burgaleta, Cancer Research, 37:1739-1742 (1977). Maisin et al., [Radiation Research, 105:276-281 (1986)] also reported that IV administration of a polysaccharide induced protection of murine hematopoietic stem cells against x-ray exposure, thereby decreasing the mortality of the mice so exposed.

Lackovic et al., [Proceedings of the Society for Experimental Biology and Medicine, 134:874-879 (1970)], took yeast cell-wall and extracted all constituent matter leaving only "mannans" that he found to be responsible for the induction of α-interferon production by monocytes. The "purified mannans" alleged to be responsible for the physiologic response had a molecular weight of 5,500-20,000 daltons.

Seljelid et al., [Experimental Cell Research, 131(1):121-129 (1981)] have observed that insoluble or gel-forming glycans activated macrophages in vitro, whereas the corresponding soluble glycans did not. Bogwald, [Scandinavian Journal of Immunology, 20:355-360 (1984)] immobilized glycans that had a stimulatory effect on the macrophages in vitro. This led the authors to believe that the spatial arrangement of the glycan was decisive for the effect on the macrophages in vitro. A purified polysaccharide isolated from Candida albicans induced an antibody response by human peripheral blood lymphocytes in vitro. Wirz et al., Clinical Immunology and Immunopathology, 33:199-209 (1984). There were significant differences between the anti-Candida antibodies in sera of normal and Candida-infected individuals. Wirz et al., supra.

The antiviral activity of polysaccharides and polysaccharides linked to peptides has been observed. Suzuki et al., Journal of Antibiotics, 32:1336-1345 (1979). Suzuki et al., supra, reported an antiviral action of peptidomannan (KS-2) extracted from mycelial culture of Lentinus edodes. Both oral and intraperitoneal administration increased the peak serum interferon titer, which protected mice against viral infections. This was different from dextran phosphate (DP-40) [Suzuki et al., Proceedings of the Society for Experimental Biology and Medicine, 149(4):1069-1075 (1975)] and 9-methylstreptimidone (9-MS) [Saito et al., Antimier. Agent & Chemotherapy, 10(1):14-19 (1976)], which induced higher titers of interferon in mice only if administered IV or IP.

Anti-inflammatory activity of Aloe vera gel has been widely reported by both oral testimonies and respected scientific journals. Rubel [Cosmetics and Toiletries, 98:109-114 (1983)] discussed fully the possible mechanism of the anti-inflammatory effect of aloe gel. Ukai et al., [Journal of Pharmacobio-Dynamics, 6(12):983-990 (1983)] noted anti-inflammatory activity of polysaccharides extracted from the fruiting bodies of several fungi. The polysaccharides demonstrated a significant inhibitory effect on carrageenan-induced edema. One of the polymers, O-acetylated-D-mannan (T-2-HN), in addition demonstrated a more marked inhibitory effect than phenylbutazone on scald hyperalgesia. Ukai et al., supra.

Other researchers have also reported anti-inflammatory effects of complex polysaccharides [Saeki et al., Japanese Journal of Pharmacology, 24(1):109-118 (1974)], glycoproteins [Arita et al., Journal of Biochemistry, 76(4): 861-869 (1974)] and sulfated polysaccharides [Rocha et al., Biochemical Pharmacology, 18:1285-1295 (1969)].

The controversy over whether the polysaccharide is a glucomannan, mannan, pectin, or of some other composition, is resolved by a series of. chemical purification steps. Yagi et al., [Planta Medica, 31(1):17-20 (1977)], using a slightly modified extraction method, isolated acetylated mannan (aloe mannan) from Aloe arborescens Miller var. natalensis. Ovodova [Khim. Prior. Soedin. 11(1):325-331 (1975)], however, earlier isolated pectin as the main component of the same aloe species.

The structure of these immunologically active polysaccharides and the types of structural variations appear to be the factors that control their potency and toxicity. Their mode(s) of action remain poorly understood; however, recent evidence indicates that several polysaccharides induce lymphocytes and macrophages to produce a wide range of immunologically active substances. For example, 2-keto-3-deoxy-D-manno-octulosonic acid (KDO) appears to be the chemical portion of lipopolysaccharide (LPS) that provides the minimum signal for macrophage host defense activation [Lebbar et al., Eur. J. Immunol. 16(1):87-91 (1986)]. The composition of the present invention possesses all of the attributes of these immunologically active substances; it is among the most potent of all known biologically active polysaccharides but differs in that no toxicity has been observed. It also manifests specific antiviral activity through alteration of viral glycoprotein synthesis.

A number of pharmacology studies have been conducted on Aloe vera gel in recent times. Results have included more rapid healing of radiation burns [Rowe, J. Am. Pharm. Assoc., 29:348-350 (1940)] and accelerated healing of wounds [Lushbaugh et al., Cancer, 6:690-698 (1953)]. Thermal burns treated with Aloe vera gel heal much faster than untreated burns [Ashley et al., Plast. Reconstr. Surg., 20:383-396 (1957), Rovatto, supra, Rodriguez-Bigas et al., J.Plast. Reconstr. Surg., 81:386-389 (1988)]. The gel is useful in treating leg ulcers [El Zawahry et al., Int. J. Dermatol., 12:68-73 (1973)] and in hastening post surgical healing (Payne, Thesis submitted to Faculty of Baylor University, Waco, TX, MS Degree). Experimental evidence suggests that extracts of Aloe vera have anti-infectious properties [Solar, Arch, Inst. Pasteur Madagascar, 47:9-39 (1979)] and enhance phagocytosis [Stepanova, Fiziol. Akt. Veshchestva, 9:94-97 (1977)].

The active fraction of Aloe vera gel has been identified by Carrington Laboratories, Inc., Irving, Texas, as a long-chain polydispersed β-(1,4)-linked acetylated mannan intersperse with O-acetyl groups having a mannose monomer-to-acetyl group ratio of approximately 1:0.91. Acemannan is the nonproprietary name of the biologically active component of Carrisyn®, a compound isolated and developed by Carrington Laboratories, Inc. See U.S. Patent No. 4,735,935, U.S. Patent No. 4,851,224, and the U.S. Patent Application Serial No. 07/229,164, and references cited therein, the disclosures of all of which are incorporated herein by reference.

Mannans, including glucomannans and galactomannans, have long been used by man. For example, galactomannans, in the form of plant gums, are widely employed as binders for control of food texture. In addition, some mannans have exhibited significant therapeutic properties (Davis and Lewis, eds. Jeanes A., Hodge J., In: American Chemical Society Symposium, Series 15. Washington, DC, American Chemical Society, 1975).

Pure mannans are relatively uncommon in higher plants, although they are a major structural component of some yeasts. For example, about 45% of the cell wall of Saccharomyces cerevisiae consists of a mannan. This mannan

is a water soluble molecule composed of β-(1,6)-, β-(1,3)-, and β-(1,2)-linked, partially phosphorylated D-mannose residues [McMurrough et al., Biochem. J., 105:189-203 (1967)]. Other biologically active mannans have been obtained from Candida utilis [Oka et al., Gann, 60:287-293 (1969), Oka et al., Gann, 58:35-42 (1968)], Candida albicans, Coccidioides immitis and Rhodotorulum rubrum [Wheat et al., Infect. Immun., 41:728-734, (1983)]. Mannans (including galactomannans and glucomannans) are relatively resistant to attack by mannosidases but can be degraded by exo- and endo-mannanases [Emi, et al., Agr. Biol. Chem., 36:991-1001 (1972), Snaith, et al., Adv. Carbohydr. Chem. Biochem., 28:401-445, (1973) Herman, Am. J. Clin. Nutr., 24:488-498 (1971), McMaster, et al., Proc. Soc. Exp. Biol. Med., 135:87-90 (1970), Jones et al., J. Biol. Chem., 243:2442-2446 (1968), Eriksson et al., Acta. Chem. Scand., 22: 1924-1934 (1968)]. The most marked biological activities of mannans in mammals are activation of macrophages and stimulation of T cells. As a result, they are potent immunostimulants with significant activity against infectious diseases and tumors [Hasenclever et al., J. Immun., 93:763-771 (1964)].

Saccharomyces mannan (15 mg/kg/day) enhances carbon clearance in normal male ddI mice, presumably acting as a reticuloendothelial system stimulant [Suzuki et al., Gann, 62:553-556 (1971)]. This same mannan also increases the number of antibody-forming cells in the spleen [Suzuki et al., Gann, 62:343-352 (1971)]. In vitro studies with mouse peritoneal cells (a mixture of macrophages and lymphocytes) indicate that some mannans and mannan-protein complexes can stimulate interferon release both in vivo and in vitro [Lackovic et al., Proc. Soc. Exp. Biol. Med., 134:874-879 (1970)]. The mannans stimulated interferon release in a manner similar to endotoxins but, in contrast to endotoxins, caused minimal toxicity (Borecky et al., Acta Virol., 11:264-266 (1967), Hasenclever, supra). The mannan from Candida albicans is active in this way, but the mannan from Saccharomyces cerevisiae is inactive [DeClercq et al., Ann. NY Acad. Sci., 173:444-461 (1970)]. Inconsistent or poor results have been obtained in other laboratories (DeClercq, supra). These differences may be due to slight structural or size differences in the polymers [Suzuki et al., Jpn. J. Microbiol., 12:19-24 (1968)]. The latter is more likely responsible since low molecular weight mannans (5.5-20 kDa) tend to be most active in the interferon-inducing assay, also Saccharomyces mannan tends to be larger than Candida mannan.

A galactomannan of 20 kDa from Lipomyces starkeyi had weak interferon-inducing properties. In contrast, Candida albicans mannan induced the appearance of interferon activity 2-24 hrs after intravenous administration (Borecky, supra).

DMG, a degraded mannoglucan from Microellobosporia grisea culture fluid, can stimulate cytotoxic activities of macrophages, natural killer (NK) cells and killer T cells, and it enhances the secretion of interleukin-1 (IL-1) and colony-stimulating factors (CSF). It has more potent antitumor activity than lentinan (a glucan from Lentinus edodes) [Nakajima et al., Gann, 75:260-268, (1984), Inoue et al., Carbohyd, Res., 114:164-168 (1983)]. DMG stimulates macrophages to produce increased amounts of IL-1. In addition, DMG enhances 1) antibody production against sheep erythrocytes, 2) natural killer activity of spleen as well as of peritoneal cells, and 3) cytostatic activity of peritoneal macrophages [Nakajima et al., Gann, 75:253-259 (1984)].

In humans, the major mannose-binding protein is an acute-phase protein; its levels rise in stressed individuals [Ezekowitz et al., J. Exp. Med., 169:185-196 (1989)]. The envelope glycoproteins of the human immunodeficiency virus (HIV gp120 and gp41) contain mannose-rich oligosaccharides that appear to be potential ligands for the mannose-binding protein. As a result, the mannose-binding protein can inhibit HIV infection of lymphoblasts and bind selectively to HIV-infected cells. Free yeast mannan can competitively interfere with binding of this protein to infected cells. Thus, factors that induce an increase in the level of the mannose-binding protein may confer protection against HIV.

## PROBLEMS TO WHICH THE INVENTION IS ADDRESSED

Virus, cancer and diseases of immune regulation continue to be major causes of both morbidity and mortality in humans, other mammals, other animals, birds, and plants. Problems associated with currently used drugs are, namely, general toxicity, lack of efficacy (or both), deficiency in specificity and development of resistance by causative organisms or agents. Hence, better non-toxic yet therapeutically efficient agents are needed for the treatment of these diseases. Acemannan has been shown to possess a unique combination of immunodulatory and antiviral properties.

## SUMMARY OF THE INVENTION

It is therefore an object to provide the use of an acetylaled mannan in the manufacture of a medicament for reducing symptoms associated with chronic respiratory diseases.

The medicament causes an effect on the respiratory system of an animal to ameliorate the symptoms associated with asthma, conjunctivitis, rhinitis and bronchitis. An acetylated mannan derivative is to be administered into the animal sufficient to cause immunomodulation of the cells and tissues responsible for the symptoms associated with asthma, conjunctivitis, rhinitis and bronchitis.

## DETAILED DESCRIPTION OF PREFERRED EMBODIMENTS

Carrisyn® is the brand name given by the assignee of the instant invention to the purified ethyl alcohol extract of the inner gel of the leaves of Aloe barbadensis Miller. The active component of Carrisyn® has been designated "acemannan" by the United States Adopted Name Council. Not less than 73% of Carrisyn® extract is acemannan; Carrisyn® extract comprises generally about 73% to 90% acemannan. Carrisyn® extract is generally produced by removing the outer sheath of the leaf, then removing and processing the inner filet or mucilage as follows: pH adjustment, ethanol extraction, freeze drying and grinding. See U.S. Application Serial No. 144,872 filed January 1988, a continuation-in-part of U.S. Application Serial No. 869,261 (now U.S. Patent No. 4,735,935). Processing in this manner predicts that essentially no covalent bonds are altered and therefore no toxic compounds are created. These manufacturing steps were developed to overcome the inability of traditional aloe product producers to standardize and stabilize the polysaccharides.

Carrisyn is a fluffy, white, amorphous powder, which is poorly soluble in water and dimethyl sulfoxide and insoluble in most other organic solvents. This powder contains not less than 73% of a polysaccharide consisting essentially of linear β(1-4)-D-mannosyl units. The polysaccharide is a long chain polymer interspersed randomly with acetyl groups linked to the polymer through an oxygen atom. The generic name for the polymer is acemannan. The degree of acetylation is approximately 0.91 acetyl groups per monomer as determined by the alkaline hydroxamate method. See Hestrin, Journal of Biological Chemistry, 180:240-261 (1949). Neutral sugars linkage analysis indicates that attached to the chain, probably through an α(1-6) linkage, is a D-galactopyranose in the ratio of approximately one for every 70 sugars. The 20:1 ratio of mannose to galactose indicates that galactose units are also linked together, primarily by a β(1-4) glycosidic bond. The chemical structure of acemannan may be represented as follows:

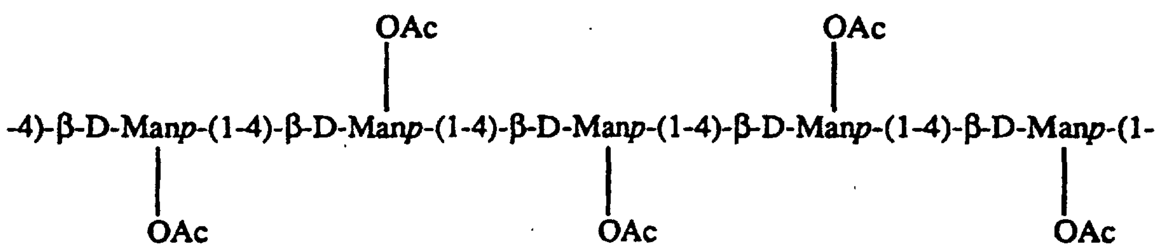

*p*=Pyranose

**General Structure of Ultrapure Acemannan**

The administration of acetylated mannan can be achieved by topical application, oral ingestion, IP route, IV route or other parenteral routes of administration.

Not only can the acetylated mannan be given to the recipient as a single agent, it can also be used in combination with other known therapeutic agents that are characterized by their requirement of the participation or aid of the host's immune system to achieve their maximal therapeutic effect.

Acemannan has now been discovered to be a potent inducer of IL-I and prostaglandin E2 (PGE2) production by human peripheral blood adherent cells in culture. The instant invention is believed to be the first practical non-toxic stimulator of IL-1 release. IL-1 is an important macrophage product reported in the literature to influence the activity and production of lymphocytes, fibroblasts, B-lymphocytes and endothelial cells. See Old,. Scientific American, 258 (5):59-60, 69-75 (1988).

IL-1 induces fibroblast proliferation which is fundamental to wound healing. IL-1 also: (1) enhances bone marrow activity; it may be therapeutic in individuals whose bone-marrow is depressed; and (2) enhances the immune system in general.

A series of experiments with mixed lymphocyte cultures (MLC) has shown that acemannan increases the alloantigenic response of these lymphocytes in a dose-related fashion. Incubation of acemannan with monocytes permitted monocyte-driven signals to enhance the T lymphocyte response to lectin. Related studies on acemannan's effects on MLC have shown an increase in phagocytosis and activity of natural killer cells. Thus, in these in vitro test systems, acemannan is non-toxic and is an immunoenhancer.

Acemannan actively stimulates lymphocytes to secrete lymphokines and also causes HIV-infected lymphocytes to produce altered glycoproteins (GP-120) by a mechanism similar to that of glucosidase I inhibitors. See

Gruters et al., Nature 330:74-77 (1987) and Pal et al., Intervirol. 30:27-35 (1989). Acemannan is phagocytized and apparently pumped to the Golgi/glycoprotein apparatus of the monocyte where it interferes directly with glycoprotein synthesis.

## A. Toxicology

The toxicological effects of acemannan have been studied in both in vivo and in vitro systems. Acemannan is not mutagenic or blastogenic in in vitro test systems. In vitro, the compound was non-toxic for H-9, MT-2 and CEM-SS lymphoid cells. In vivo toxicology studies on acemannan include a 91-day subchronic oral toxicity study in dogs, a 180-day chronic oral toxicity study in rats and an 180-day chronic oral toxicity study in humans. In these studies, no toxic effects were noted in dogs receiving up to 825 mg/kg of acemannan per day for 91 days. No clinical, gross pathologic or toxic effects were noted in rats receiving up to 38,475 ppm acemannan in their feed for 180 days. No adverse clinical or toxic effects were noted in human patients receiving 800 mg per day of acemannan for 180 days.

In pilot studies, administration of acemannan to dogs caused an absolute monocytosis in blood samples taken for complete white blood cell counts and morphology differential. Within 2 hours after oral administration of high doses of acemannan, large activated monocytes appeared in circulation. A similar effect has been observed in humans.

A study was performed using human peripheral blood monocyte cell cultures and $^{14}C$-labeled acemannan to track the incorporation or absorption of acemannan into a biological system. In this study, detectable amounts of $^{14}C$-labeled acemannan were absorbed or ingested by human peripheral monocyte/macrophage cells. Peak incorporation occurred at 48 hours. At a concentration of 5 mg/ml, the $^{14}C$-labeled acemannan was not cytotoxic to the monocyte/macrophage cells, and the weight/volume (w/v) digested cell mass was 760 times greater than the w/v of the digested acemannan solution. These results suggest that the macrophage is capable of maintaining intracellular concentration of acemannan at very high levels that are not cytotoxic.

A pyrogen assay was performed in rabbits in accordance with the pyrogen test protocol outlined in the U.S. P. XXI, Biological Test [151], using a 1 mg/ml injectable solution of acemannan. More frequent temperature measurements were taken than specified in the U.S.P. because of the unknown systemic effects of injected acemannan. Temperature changes in test animals did not exceed minimum changes allowed by the U.S.P. protocol; therefore, the solution met the U.S.P. requirements for absence of pyrogens. Acemannan injectable elicited a maximum body temperature increase of 0.3°C in one rabbit. This temperature rise occurred 90 minutes after injection. Acemannan is an inducer of IL-1 secretion by macrophages and monocytes in vitro. Since IL-1 is a potent pyrogen, this might explain the minimal, delayed temperature rise in this rabbit.

Twenty-four human subjects enrolled in and completed the study of the safety and tolerance of orally-administered acemannan. Clinical laboratory results showed that shifts out of the normal range occurred in the following: $CO_2$ in seven subjects, cholesterol in three subjects, triglycerides in two subjects, phosphorous in one, hemoglobin in four, basophils in two, monocytes in three, eosinophils in three, lymphocytes in four, neutrophils in two, and one each in red and white blood cells. Small numbers of red and white blood cells were also found in the urine. None of these shifts was clinically relevant.

Immune profile results showed group differences between Day 1 to Day 7 values for the following: CD-16, CD-4 (T-4), CD-8+Leu7, CD-4+CD-25, CD-8+CD-16, Leu7 and TQ-1. Mitogen responses were in the low range.

Vital signs did not appear to exceed normal ranges. There were no group differences in urine output. One subject in Group IV had diarrhea and loose stools during the study. One subject in Group I had loose stools during days 2 to 4 of the study. A total of 5 subjects reported a total of eight adverse events. All the events occurred in subjects receiving 1600 or 3200 mg oral acemannan daily for 6 days.

## B. Mode of Administration

The physical properties of acemannan allow it to be formulated and incorporated into all pharmaceutical dosage forms known to those skilled in the art. The biopharmaceutical and toxicological properties of acemannan permit it to be used in tissues and organs of living organisms and to be administered over a wide range of doses.

Acemannan may be administered to an animal orally, parenterally, topically and locally, in a daily dosage of 0.001 mg/kg to 1000 mg/kg body weight per day.

Mixed with suitable auxiliaries, acemannan may be compressed or filled into solid dosage units such as pills, tablets and coated tablets, or it may be processed into capsules. These oral dose forms would be administered at a dosage of about 0.1 mg/kg to 1000 mg/kg of body weight per day.

By means of suitable liquid vehicles, acemannan can be injected in solutions, suspensions or emulsions. These products would be administered at a rate of 0.001 mg/kg to 1000 mg/kg of body weight per day. As an adjunctive component of a vaccine or other product, acemannan would be used at a rate of 0.001 to 1000 mg per unit dose of adjuvanted product.

Topical administration of acemannan can be in the form of a processed gel, cream, lotion, solution, ointment or powder. These formulations could contain up to 90% acemannan.

**Example 1**

**PRODUCTION OF INTERLEUKIN-1 AND PGE2 BY HUMAN ADHERENT PERIPHERAL BLOOD LEUKOCYTES STIMULATED WITH ACEMANNAN**

**A. Induction of IL-1 Production**

Human mononuclear cells were separated from heparinized whole blood by density-gradient centrifugation in Ficoll-Hypaque (Pharmacia, Sweden). After washing, cells were resuspended at a concentration of 2 x $10^{-6}$ cells/ml in RPMI-1640 with 25 mM Hepes, and supplemented with 50 U/ml penicillin, 50 µg/ml streptomycin and 2 mM L-glutamine. Two ml aliquots of the cell suspensions were dispensed into each well of a six-well plate and incubated for 1 hour at 37°C in a 5% $CO_2$-humidified atmosphere. After removal of nonadherent cells, adherent cells were washed three times with the medium described above. Two ml of medium supplemented with 5% pooled human AB serum were added to each well. Cultures were stimulated with acemannan at different concentrations. Simultaneous controls with lipopolysaccharide (LPS) from E. coli (Sigma 0111:B4) at a final concentration of 20 µg/ml, and without any addition (background), were included. The cultures were incubated at 37°C as described above for 24 hours. Supernatants were harvested, centrifuged to remove cells, and dialysed against 500 volumes of PBS for 48 hours (changed once), followed by 4 hours of dialysis against 20 volumes of RPMI-1640 with 25 mM Hepes, antibiotics and L-glutamine as described. Supernatants were frozen at -20°C until IL-1 activity was evaluated.

**B. IL-1 Determination in Supernatants**

Two different procedures were used to assay IL-1: (1) the thymocyte proliferation assay and (2) an ELISA assay specific for IL-1.

1. Thymocytes from $C_3H$/HeJ mice 5-8 weeks old were used. A homogeneous cell suspension was prepared in minimum essential medium (MEM) supplemented with 5% FCS, 100 U/ml penicillin, 50 g/ml streptomycin, 2 mM L-glutamine and 5 x $10^{-5}$ M 2-mercaptoethanol. The cell concentration was adjusted and dispersed into 96-well plates at 1 x $10^6$ cells/well. Phytohemagglutinin (PHA) was added to each well at a concentration of 10 µg/well. Samples were diluted serially and a volume of 25 µl was added to each well, starting from 1:10 to the final dilution. Every dilution was tested in quadruplicate. Plates were incubated at 37°C in a humidified atmosphere with 5% $CO_2$ for 72 hours and were pulsed with [$^3$H]-thymidine (0.5 µCi/well) during the last 16 hours. Cells were harvested onto fiberglass filters with an automatic cell harvester, and radioactivity was measured by standard scintillation procedures. Results are represented as cpm of thymidine incorporation by thymocytes in response to the supernatants at a final 1:10 dilution.
2. Two-site "Sandwich" ELISA for IL-1. This procedure has recently been described in Journal of Immunology, 138:4236 (1987), the disclosure of which is hereby specifically incorporated herein by reference. See also U.S. Patent No. 3,654,090 and U.S. Patent No. RE 31,006 to Schuurs et al. Briefly, monoclonal purified antibody IL-1-H6 against IL-1β, (100 µl/well, 10 µg/ml) was coated on vinyl assay plate wells overnight at 4°C. The wells were washed with PBS/0.5% Thimerosal and countercoated with 200 µl of 5% non-fat dry milk/0.5% Thimerosal/PBS for 1 hour at room temperature. After washing, 50 µl/well of sample or human recombinant IL-1 standard and 50 µl of another monoclonal antibody against a non-overlapping epitope of IL-1, [biotinylated IL-1β-H67 (2 µg/ml) in 1% non-fat dry milk/0.5% Thimerosal/PBS], were added, and the plates were incubated for 2 hours at room temperature. After washing, 100 µl/well of a 1:1000 dilution of streptavidin-peroxidase were added and the plate was incubated for 1 hour. The wells were washed, incubated for 30 minutes in the dark with 100 µl OPD substrate solution, and absorbance at 450 nm was measured.

**C. Determination of PGE2**

$PGE_2$ was evaluated with a radioimmunoassay in the same non-dialyzed supernatants. The antibody to PGE2 (ICN Biomedical, Inc., Costa Mesa, CA) was used according to the manufacturer's instructions, which are incorporated herein by reference.

## D. Observations

Representative experiments are shown in Table 2. Acemannan is a potent inducer of IL-1 production by human adherent peripheral blood leukocytes. At doses between 1 and 10 µg/ml, acemannan extract induced production of IL-1 comparable to that induced by 20 µg/ml LPS, which is the reference inducer of IL-1 production. Acemannan in the same dose range also induced the production of PGE2 at levels comparable to those induced by 20 µg/ml LPS (positive control).

Table 2

| INDUCTION OF $PGE_2$ SYNTHESIS BY HUMAN PERIPHERAL BLOOD ADHERENT CELLS STIMULATED BY ACEMANNAN AND BY LIPOPOLYSACCHARIDE (LPS). | | |
|---|---|---|
| **Experiment No.** | **Stimulator** | **PGE2 (ng/ml)** |
| 198 | 0 | 0 |
| | LPS 20 µg/ml | 2.6, 3.9 |
| | Acemannan 10 µg/ml | 3.5 |
| | Acemannan 1 µg/ml | 0 |
| 148 | 0 | 0 |
| | LPS 20 µg/ml | 0.5, 1.3 |
| | Acemannan 10 µg/ml | 0.7 |

### Example 2

### EFFECT OF ACEMANNAN ON PHAGOCYTOSIS IN VITRO

The effect of acemannan was studied in vitro to ascertain its effect on phagocytic function. CBA mice were injected IP with 1 mg/kg acemannan, and peritoneal and splenic macrophages were collected 3 days later. Thioglycolate and saline were similarly tested as positive and negative controls, respectively. The macrophages were incubated with sheep red blood cells (SRBC) as ingestion particles in the presence and absence of anti-SRBC titers, and phagocytosis was measured histologically as percent cells that ingested SRBC. Although non-specific phagocytosis was increased slightly after acemannan treatment, phagocytosis was significantly increased in the presence of antibody. In the presence of complement, acemannan-stimulated, antibody-mediated phagocytosis was increased to an even greater extent. These results indicate that acemannan may increase the number of macrophages and enhance their phagocytic activity. Such responses may contribute to acemannan's effectiveness as a stimulant of wound healing and as an anti-infectious agent.

## A. Methods and Materials

Acemannan was stored at room temperature in its dried form. The amount needed for each experiment was weighed out and microwaved in 2-minute exposures at 600 watts of power. It was then transferred to a sterile plastic centrifuge tube and microwaved for 1 additional minute. The material was diluted in cell culture medium (RPMI-1640) to the desired concentration.

Phagocytic Cells: Mouse spleen cells were obtained from BALB/c mice purchased from Harlan Sprague-Dawley. The mice were killed by $CO_2$ asphyxiation, and their spleens were removed aseptically. Subsequently, the cells were separated into adherent and non-adherent populations by nylon wool column fractionation according to the method of Journal of Immunology, 71:220, the disclosure of which is hereby specifically incorporated by reference. Adherent cells were determined by microscopic analysis, as described below, to be macrophages (monocytes) and lymphocytes in a ratio of 4 to 1. After single-cell suspensions were obtained by monolayer disruption, both adherent and non-adherent single cell preparations were placed on ficoll-hypaque and centrifuged to obtain a mixture of lymphocytes and macrophages.

Blastogenesis Assay: A standard blastogenesis assay was set up as outlined below. The mitogen used in the assay was PHA-P obtained from Burroughs Wellcome. As indicated for individual experiments, the cultures were maintained for 72 hours in a 5% $CO_2$, humidified atmosphere. Tritiated thymidine was added during the last 6 hours of the culture. Cell concentrations per well, using flat bottom microtiter tissue culture plates, were 5 x $10^5$ mouse cells/0.2 ml. Cells were deposited in the wells and acemannan or mitogen was added. A stimulation index (SI) was calculated using the formula:

$$SI = \frac{\text{cpm experimental - cpm background}}{\text{cpm control - cpm background}}$$

Cell Staining: Briefly, smears of cells were stained by non-specific esterase stain as follows. Approximately 2 x $10^6$ cells in 2 drops were mixed with 2 drops of fetal calf serum and 4 drops of a fixative solution consisting of a mixture of 25 ml of 35% formaldehyde, 45 ml of acetone, 100 mg of $KH_2PO_4$, 10 mg of $Na_2HPO_4$ and 30 ml of water. The slides were incubated with a mixture of 10 mg of naphthyl acetate and 4.5 mg of Fast Blue stain in 1.4 ml of ethylene glycol monomethyl ether with 5 ml of 0.1 M Trismaleate buffer, pH 7.8 (Wright's stain). The stain was allowed to react for 10 minutes, then washed in water for 20 seconds. A counterstain of 0.2 g of Giemsa stain, 12.5 ml of ethanol and 12.5 ml of glycerol was used for 30 seconds before washing again.

Induction of Peritoneal Macrophage Cells: Saline thioglycolate broth (1 mg/kg) or acemannan (1 mg/kg) was injected IP into female BALB/c mice to induce peritoneal exudate macrophage cells. Induced cells were removed from the peritoneal cavity 3 days post-injection.

Macrophages were washed twice with phosphate-buffered saline (PBS) and covered with 2 ml of fresh medium; 0.1 ml of the macrophage suspension was added to each tube. Cultures were placed for 30 to 60 minutes into a 37°C, humidified 5% $CO_2$-95% air incubator. Cultures were washed twice with PBS and covered with 2 ml of PBS. One of each pair of coverslips was removed with needle-nosed forceps, dipped for 5 seconds only in distilled water, and promptly replaced in the culture dish. The PBS was removed, and the cultures were covered with ice-cold glutaraldehyde. After 10 minutes, the glutaraldehyde was removed, and the coverslips were overlaid with distilled water.

Mounted coverslips were examined promptly with the oil immersion lens of a phase contrast microscope. Attachment was scored on the coverslip that was not subjected to hypotonic shock, whereas ingestion was scored on the coverslip that was lysed in distilled water.

Antibody-Dependent and Antibody-Independent Phagocytosis: SRBC, obtained from Austin Biologics Laboratory, Austin, Texas, were washed three times in PBS (pH 7.2). BALB/c mice were given IP injections of $10^6$ cells and bled on day 14 post-injection. Serum was collected, pooled and heat inactivated at 56°C for 45 minutes. Agglutination titers were determined to be 1024 using round-bottomed microtiter wells.

Antibody-independent phagocytosis was determined by incubation of SRBC (0.5% v/v) with macrophages ($10^6$) in RPMI-1640 containing 20% fetal calf serum (FCS). Slides were prepared at various intervals and stained. The percent macrophages that had ingested red cells was determined visually by counting 200 cells/slide and three slides/animal.

Antibody-dependent phagocytosis was determined using SRBC (0.5% in RPMI-1640 with 20% FCS) mixed with anti-SRBC serum or IgM fraction (minimum titer of 2000). The mixture was incubated for 15 minutes at 37°C, then washed twice in PBS (pH 7.2) and resuspended to the original volume.

Serum Fractionation: Whole serum was fractionated to remove IgM by euglobulin precipitation and dialysis against distilled water. After dialysis at 4°C for 24 hours, precipitate was removed by centrifugation at 1500 x G for 20 minutes, and supernatant was analyzed by ion electrophoresis and complement-mediated lysis. Less than 5% of the original IgM remained.

### B. Results

To evaluate the effect of acemannan on macrophages, the first experiment utilized mouse spleen cells cultured in vitro with acemannan (Table 3).

Table 3

| PERCENT CELL TYPES BY HISTOLOGICAL EVALUATION OF MOUSE SPLEEN CELLS IN CULTURE | | | | | |
|---|---|---|---|---|---|
| **Time in Culture** | **Cells(a)** | **Acemannan (μg/well)** | | | |
| | | **0.0** | **0.002** | **0.02** | **0.2** |
| 72 hours | macrophages | 30±6 | 32±7 | 41±3 | 45±9 |
| | lymphocytes | 70±5 | 68±8 | 59±3 | 55±6 |
| 96 hours | macrophages | 22±4 | 28±4 | 36±6 | 38±8 |

(a) Macrophages (monocytes) were determined by esterase staining. The results are expressed as mean ±S.D. The results are from six experiments with 200 cells studied/experiment. "Lymphocytes" are cells that did not stain by esterase and had the appearance of lymphocytes by Wright's stain.

Table 3 (continued)

| PERCENT CELL TYPES BY HISTOLOGICAL EVALUATION OF MOUSE SPLEEN CELLS IN CULTURE | | | | | |
|---|---|---|---|---|---|
| Time in Culture | Cells(a) | Acemannan (μg/well) | | | |
| | | 0.0 | 0.002 | 0.02 | 0.2 |
| | lymphocytes | 78±8 | 72±7 | 64±10 | 62±4 |

(a) Macrophages (monocytes) were determined by esterase staining. The results are expressed as mean ±S.D. The results are from six experiments with 200 cells studied/experiment. "Lymphocytes" are cells that did not stain by esterase and had the appearance of lymphocytes by Wright's stain.

Cultures were incubated for 72 or 96 hours, and at termination of the experiment smears were made and stained by Wright's stain and by the esterase method. The relative percentage of macrophages and lymphocytes was determined. At 72 hours there was a dose-related increase in macrophage numbers from 30% with no acemannan to 45% with 0.2 μg of acemannan per well. Since data are expressed as percent cells, there was a concomitant reduction in lymphocytes. At 96 hours there was also a dose-related increase in the percentage of macrophages in the presence of acemannan. At 96 hours, the cultures with 0.2 μg of acemannan per well showed significant acidosis, as indicated by a yellow coloring. Furthermore, 96-hour cultures had a lower percentage of macrophages, possibly due to the longer time in culture. To relate the acemannan-induced increase in macrophage numbers to a known standard, a similar experiment was conducted with the mitogen PHA-P. Results are shown in Table 4.

Table 4

| PERCENT CELL TYPES BY HISTOLOGICAL EVALUATION OF MOUSE SPLEEN CELLS IN CULTURE | | | | | |
|---|---|---|---|---|---|
| Time in Culture | Cells(a) | PHA-P (μg/well) | | | |
| | | 0.0 | 0.02 | 0.01 | 0.2 |
| 72 hours | macrophages | 33±8 | 32±6 | 30±6 | 31±5 |
| | lymphocytes | 70±12 | 68±8 | 70±6 | 69±4 |
| 96 hours | macrophages | 18±6 | 21±3 | 26±6 | 25±5 |
| | lymphocytes | 77±10 | 79±4 | 74±8 | 75±6 |

(a) Monocytes were determined by esterase staining. The results are expressed as mean ±S.D. The results are from six experiments. "Lymphocytes" are cells that did not stain by esterase and had the appearance of lymphocytes by Wright's stain.

Although the percentage of macrophages did not change at 72 hours, there was a dose-related increase in macrophages after incubation with PHA-P for 96 hours. By comparison, acemannan was twice as effective as PHA-P. The percentage of macrophages increased a maximum of 16 with acemannan compared to 7 with PHA-P (Tables 3 and 4).

Since acemannan appeared to increase the percentage of macrophages, it was decided to determine whether the activity of the phagocytes was also increased. Peritoneal exudate cells from CBA mice given saline, thioglycolate broth or acemannan were used [as phagocytes] with sheep red blood cells as the particles to be ingested (Table 5).

Table 5

| NONSPECIFIC PHAGOCYTOSIS OF SHEEP ERYTHROCYTES BY PERITONEAL EXUDATE(a) | | | | | | |
|---|---|---|---|---|---|---|
| | Percent of Phagocytosis(b) Time (minutes) | | | | | |
| Trmt. | 0 | 5 | 10 | 20 | 60 | 120 |
| Saline | 3±3 | 11±6 | 15±10 | 25±9 | 45±12 | 52±15 |

(a) The results were determined by counting 200 cells/slide with two slides/animal. The results are based on two experiments.

(b) Percent phagocytosis indicates the proportion of cells showing erythrocyte ingestion. The results are expressed as mean ±S.D.

Table 5 (continued)

| NONSPECIFIC PHAGOCYTOSIS OF SHEEP ERYTHROCYTES BY PERITONEAL EXUDATE(a) | | | | | | |
|---|---|---|---|---|---|---|
| | Percent of Phagocytosis(b) Time (minutes) | | | | | |
| **Trmt.** | **0** | **5** | **10** | **20** | **60** | **120** |
| Thio. | 1±1 | 14±8 | 20±8 | 52±14(c) | 84±32(c) | 89±21(c) |
| Ace. | 3±2 | 10±6 | 12±8 | 41±18 | 61±18 | 63±23 |

(a) The results were determined by counting 200 cells/slide with two slides/animal. The results are based on two experiments.

(b) Percent phagocytosis indicates the proportion of cells showing erythrocyte ingestion. The results are expressed as mean ±S.D.

(c) Significantly different from saline control group, assessed by the Student's t-test at the 95% confidence level.

Over a 120-minute period, nonspecific phagocytosis increased from 3% to 52% in saline controls, whereas percent phagocytosis in cells from thioglycolate broth-treated animals rose to 89%. Phagocytosis in acemannan-treated animals rose to 63% at 120 minutes. Acemannan-stimulated phagocytosis was greater than that in controls after 20-120 minutes; however, the differences were not statistically significant.

To determine whether the acemannan effect on phagocytosis was antibody-dependent, a similar experiment was performed with anti-SRBC (Table 6).

Table 6

| ANTIBODY MEDIATED PHAGOCYTOSIS(a) | | | | | |
|---|---|---|---|---|---|
| **Phagocyte Antihody Titer (x 103)(b)** | | | | | |
| **Source** | **Pretreatment** | **0** | **2** | **4** | **8** |
| Peritoneum | Saline | 15±8 | 43±10 | 39±9 | 19±11 |
| | Thioglycolate | 49±11 | 89±22 | 80±22 | 58±14 |
| | Acemannan | 36±14 | 73±13(c) | 62±8 | 40±13 |
| Spleen | Saline | 11±4 | 38±9 | 32±11 | 20±4 |
| | Thioglycolate | 29±9 | 73±13 | 54±16 | 38±12 |
| | Acemannan | 21±10 | 60±9(c) | 51±17 | 26±11 |

(a) Phagocytosis is expressed as the mean % of cells showing erythrocyte ingestion ±S.D.

(b) The antibody titer by agglutination was shown to be 1:1024. Pre-treatment and cell sources are discussed in Methods.

(c) Significantly different from saline control, assessed by the Student's t-test at the 95% confidence level.

Sera were inactivated with heat (56°C for 30 minutes), and the antibody titer used was 2 x $10^3$, well above the hemagglutination titer. In this experiment, macrophages were obtained from two sources, the peritoneal cavity and the spleen. Again, mice were pretreated with IP injections of 1 ml of 0.9% saline, 1 mg/kg thioglycolate or 1 mg/kg acemannan. At a titer of 2 x $10^3$, the phagocytic activity of thioglycolate-induced peritoneal macrophages was twice as great (89% vs. 43%) as activity from the saline-induced controls, whereas acemannan-induced macrophages were more active by 30% (73% vs. 43%) compared to controls. The difference between phagocytic activity in the acemannan-treated and saline control groups was statistically significant.

Similar results were seen with macrophages obtained from mouse spleens. Phagocytic activity was lower than that of macrophages obtained from the peritoneal cavity, possibly due to manipulations of the spleen cells. Again, at a titer of 2 x $10^3$, acemannan-induced macrophages were significantly higher in phagocytic activity than saline controls at the 95% confidence level; phagocytic activity was similar to control at a titer of 8 x $10^3$.

To determine the effect of complement (C') on antibody-mediated phagocytosis, an experiment utilizing addition of C' to media was undertaken. (Table 7).

Table 7

| COMPARISON OF COMPLEMENT-MEDIATED PHAGOCYTOSIS | | | |
|---|---|---|---|
| | | % Phagocytosis(a) | |
| Cell Source | Phagocyte Inducer | +C' | -C' |
| Peritoneum | Saline | 24±11 | 18±9 |
| | Thioglycolate | 84±10 | 62±12 |
| | Acemannan | 70±8(b) | 54±4 |
| Spleen | Saline | 18±11 | 16±9 |
| | Thioglycolate | 54±9 | 41±11 |
| | Acemannan | 48±10 | 35±6 |

(a) Phagocytosis is measured as percent uptake of sheep erythrocytes ±S.D. after incubation for 30 minutes. Guinea pig complement was added.
(b) Significantly different compared to -C', assessed by the Student's t-test at the 95% confidence level.

To assure that lysis would not occur, IgM-depleted mouse serum was used (see Methods). The titer utilized was 3000, as determined by hemagglutination and the Coombs technique. Cells from both the peritoneal cavity and spleen were more active in phagocytosis with the addition of C' than without C', although the difference was statistically significant only with peritoneal cells induced by acemannan.

Finally, an experiment was performed to differentiate the effect of acemannan phagocytosis and adherence (Table 8).

Table 8

| COMPARISON OF PHAGOCYTOSIS AND ADHERENCE(a) | | | |
|---|---|---|---|
| Cell Source(b) | Pre-treatment | Phagocytosis | Adherence |
| Peritoneum | Saline | 5±8 | 6±4 |
| | Thioglycolate | 12±9 | 23±9(c) |
| | Acemannan | 11±9 | 18±10(c) |
| Spleen | Saline | 8±7 | 14±11 |
| | Thioglycolate | 14±6 | 36±10(c) |
| | Acemannan | 10±8 | 20±7(c) |

(a) Cell mixtures were allowed to incubate for 7 minutes.
(b) Results are reported as percent phagocytes showing phagocytosis or adherence ± S.D. The results are from one experiment with 200 cells scored/animal with three animals used.
(c) Significantly different from saline controls, assessed by the Student's t-test at the 95% confidence level.

In this experiment, antibody to SRBC was used in a titer of 2,000, but the experiment was stopped after 7 minutes. Acemannan-induced macrophages from both the peritoneum and spleen were more efficient in adherence than the saline controls and, as seen previously, less efficient than the thioglycolate-induced group.

## C. Discussion

The results indicate that acemannan both directly and indirectly stimulates phagocytosis. The results also indicate that acemannan enhances phagocytosis by macrophages, both non-specifically and specifically, through antibody-mediated reactions. This demonstrates that acemannan has immunostimulatory properties on phagocytes.

## Example 3

### ENHANCEMENT OF ALLO-RESPONSIVENESS OF HUMAN LYMPHOCYTES BY ACEMANNAN

This example was designed to test the capacity of acemannan to enhance immune response to alloantigen and to test whether the potential enhancement is a monocyte-driven phenomenon. Acemannan did not enhance lymphocyte response to syngeneic antigens in the mixed lymphocyte culture (MLC), but, importantly, it increased alloantigenic response in a dose-response fashion ($2.6x10^{-7}$-$2.6x10^{-9}$M). This effect of acemannan was shown to be a specific response and to concur with concentrations of in vitro acemannan achievable in vivo. A separate series of mixing experiments demonstrated that acemannan incubation with monocytes permitted monocyte-driven signals to enhance T cell response to lectin. It is concluded that acemannan is the active ingredient of the Aloe vera plant and is an important immunoenhancer in that it increased lymphocyte response to alloantigen. It is suggested that the mechanism involves enhancement of monocyte release of IL-1 under the aegis of alloantigen. This mechanism may explain in part the capacity of acemannan to abrogate viral infections in experimental animals and man.

This example was designed to directly assess the impact of acemannan as an immune enhancer in the model of monocyte-T-lymphocyte, cell-cell interaction response to alloantigen presented in the mixed lymphocyte culture. This model tests the capacity of acemannan to stimulate additional monocyte-macrophage functions in an immunologically relevant model.

**A. Materials and Methods**

1. Cell Preparation. Mononuclear leukocytes were obtained from the peripheral blood of normal, informed and consenting human volunteers under the aegis of a study approved by the Institutional Review Board of the University of Texas Southwestern Medical Center at Dallas. Peripheral blood was diluted 1:3 in Hanks' balanced salt solution (HBSS) and layered on top of a ficoll-hypaque gradient. Cells from subjects known to be major histocompatibility disparate were obtained on each study day to ensure a positive mixed lymphocyte reaction. For specific experiments, more carefully characterized pedigrees of cells which inhibit the mononuclear leukocyte pool were isolated. T-lymphocytes were isolated by the standard nylon wool separation technique. The nylon effluent cells contained about 90% pure T cells. T-8 lymphocytes and monocyte-macrophages preferentially adhere to the column. The adherent population was removed by forcibly pushing media through the column with a plunger. To enrich for monocytes (macrophages), the glass adherence procedure was utilized to produce a population greater than 95% pure.

2. Acemannan. Acemannan was tested in these studies by preparing a 0.5% (w/v) solution in RPMI-1640 medium and further diluting to the following working concentrations:

$$2.6 \times 10^{-7}M, 2.6 \times 10^{-8}M \text{ and } 2.6 \times 10^{-9}M.$$

3. Mixed Lymphocyte Cultures (MLC). Unidirectional MLC were set up in microtiter, flat-bottom tissue culture plates (Costar Co., Cambridge, MA). Mononuclear cells, isolated by the ficoll-hypaque density gradient technique discussed above, served as stimulator cells after exposure to 2000 rads for 30 minutes in a cesium source (Gammacell, Atomic Energy of Canada, Ontario, Canada). Responder cells that had been similarly isolated and stimulators were adjusted to $1.3x10^6$ cells/ml. To each well the following were added: 25 μl of acemannan or media (control), 25 μl of RPMI-1640 supplemented with 10% fetal bovine serum and 75 μl of each cell population. Cells were incubated at 37°C in 5% $CO_2$: 95% air for 6 days. Cultures were pulsed with 25 μl of $^3$H-thymidine (1 μCi/well) for 4 hours, after which the cells were harvested and counted. To test the specificity of acemannan on the afferent recognition and response to MLC, additional unidirectional MLC were set up with the agent added just 20 minutes before the cells were pulsed with $^3$H-thymidine.

4. Monocyte-T Cell Interaction. Lewis female rat spleens were teased through a sterile steel mesh into RPMI-1640 medium. Mononuclear leukocytes were collected from the interface of a ficoll-hypaque density gradient as described above. Monocytes, obtained by enrichment on glass petri dishes and adjusted to a final concentration of $10^6$/ml, were incubated with varying doses of acemannan or medium (control) in a total volume of 2 ml and incubated for 24 hours at 37°C. The monocytes were harvested, extensively washed with fresh medium and co-cultured with syngeneic T lymphocytes at a ratio of 10 T-cells:1 monocyte, with the plant lectin phytohemagglutinin (Difco, Detroit, MI) (1:100) for 48 hours at 37°C. Cells were harvested over a MASH II (Whittaker, MA Bioproducts, Walkersville, MD), placed in fluor and counted in a scintillation counter (Beckman Laboratories, Chicago, IL). A control experiment was performed by incubating T lymphocytes with acemannan, followed by wash and co-culture with freshly prepared T lymphocytes, again at 10:1 along with PHA-P.

## B. Results

1. Alloantigenic Response. Acemannan had no statistically important effects on the response of T-cells to autoantigens. When the agent was added at the beginning of MLC, cells receiving syngeneic stimulation incorporated tritiated thymidine equally in the presence or absence of test reagent at the doses described. In the absence of oral acemannan these MLC incorporated 2616±1099 cpm of tritiated thymidine at the end of a 4 hour pulse. Although there was a trend upward with respect to the dose of agent added (3281±1355 at 2.6 x $10^{-9}$M, 3742±1670 at 2.6 x $10^{-8}$M, and 3828±1978 at 2.6 x $10^{-7}$M), none of these rates of isotopic incorporation into DNA was different to a statistically significant degree.

In contrast to the absence of effect of acemannan on autoresponse in the MLC was the agent's effect on alloresponse in the same immunologic assay. First, acemannan did not interfere with the capacity of lymphocytes to recognize and respond to class II alloantigenic differences in the MLC; this was apparent when the syngeneic cultures were compared to the allogeneic response in the presence of the lowest concentration of drug. Second, there was a dose-response-related enhancement of alloresponse by acemannan such that the culture treated with the highest dose, 2.6 x $10^{-7}$M, reflects a nearly 60% increase over the non-acemannan culture. The dose response relationship is most convincingly demonstrated as the enhanced allogeneic response is shown to be significant for each dose of acemannan tested with respect to the no acemannan condition.

To ascertain whether acemannan exerts a specific effect on lymphocyte alloresponse or a nonspecific effect on tritiated thymidine incorporation, the reagent was added at the conclusion of a 7 day mixed lymphocyte culture MLC, 20 minutes before addition of the tracer to the culture. There was no effect of acemannan when added in this manner as a pulse at the conclusion of the MLC. These data support the specificity of the acemannan effect on enhancement of lymphoid response in the MLC.

2. Acemannan and Monocyte-T Cell Cooperation. To test the hypothesis that acemannan directly stimulates the monocyte responding to alloantigen to provide signal(s) to enhance lymphoid response to antigen and/or mitogen, purified populations of monocytes were incubated for 24 hours with various doses of acemannan. At the conclusion of the incubation the cells were washed extensively and then co-cultured with T lymphocytes at a ratio of 10:1, to simulate the natural ratio found in peripheral blood. Co-cultured cells were stimulated with phytohemagglutinin. The co-cultures with monocytes that were previously incubated with acemannan had a significantly increased mitogenic response in a dose-related fashion.

## C. Discussion

This example has explored the capacity of acemannan to function as an immunostimulating drug with important clinical consequence.

Acemannan is believed to be capable of limiting DNA and retrovirus infections that cause significant diseases in animals and in man. For example, in an animal model, acemannan ameloriated feline viral rhinotracheitis. Additional evidence shows that acemannan in vitro and in vivo may be effective against Herpes simplex II virus, the measles virus, and perhaps HIV. Evidence indicates that the immunological mechanism may involve enhancement of the monocyte, both as a phagocytic cell and as a cell that contributes to afferent recognition of antigen. Studies have shown direct enhancement of phagocytic properties of the monocyte, on the one hand, and an increase in the absolute numbers of that important cell, on the other. Mounting evidence supports the concept that acemannan enhances the elaboration by the activated monocyte of the signal substance IL-1.

The studies described in this example were directed specifically at exploring the mechanism by which acemannan may be an immuno-enhancing reagent. Mixed lymphocyte cultures are in vitro models of the manner in which immunocompetent cells participate in response to antigen of the variety that is necessary for recognition and response to virus. In this reaction, there are important monocyte-T-lymphocyte interactions that generate a response to alloantigen. It was this model that was chosen for testing the capacity of the acemannan to function as an immunoactivator.

Acemannan is therefore an important enhancer of the alloantigenic response in MLC. There is a dose-response relationship with enhancement at the highest dose tested of about 60% above basal. This represents not only a statistically significant but also a biologically relevant increase in response to alloantigen and may serve as one means by which the drug can aid the response of the organism to viral assault. This effect of acemannan was shown to be specific for the allogeneic stimulus, provided the drug did not enhance either basal response to self (syngeneic MLC) or non-specific incorporation of a tracer DNA precursor, tritiated thymidine, when drug was added at the conclusion of the MLC.

A second series of experiments tested the hypothesis that monocyte-T-lymphocyte interactions may be, at least in part, responsible for the heightened alloresponse in the MLC. In this series of experiments acemannan was

incubated along with monocytes, after which the treated, extensively washed monocytes were mixed with freshly prepared, syngeneic T-lymphocytes that had not been exposed to and would not be exposed to acemannan. These experiments demonstrate the enhancement of T-lymphocyte response to the polyclonal mitogen phytohemagglutinin at a magnitude equal to the response that had been seen previously in the MLC--approximately 55% above baseline and dose-response relationship.

The lowest dose that was tested in the study that was effective in the MLC had no effect in the monocyte experiment. It is not surprising that the threshold dose may be different for the two models tested, polyclonal response to mitogen and alloantigenic response in the MLC. It can also be observed that the monocyte experiment is a more stringent test of the effect of acemannan because it presents a treated cell type, the monocyte, to T cells that then see an immune stimulus in the absence of the drug. While the alloantigenic response may be due solely or in great measure to acemannan-enhanced monocyte production of IL-1, the lesser polyclonal mitogen-enhanced response may be a consequence of an assay of immune stimulations, each with a different threshold response to acemannan.

The dose of acemannan used in these experiments is clinically relevant. The dose range selected was chosen precisely to bracket that concentration of acemannan that could be expected to be achieved in plasma if the drug distributes in extracellular water and is absorbed at the rate of a third of the orally-administered dose, figures that were based on previous pharmacologic studies in dogs. The actual concentrations achievable in man have also been shown to be in this range, further supporting the potential relevance of these studies for clinical practice.

Acemannan was shown by these experiments to cause monocytes to release monocyte-driven signals to enhance T4 cell response to lectin. While acemannan did not enhance lymphocyte response to syngeneic antigens in MLC, it did increase MLC alloantigenic response in a dose-related manner. This response was shown to be an acemannan-specific response at acemannan concentrations achievable in vivo.

This experimental documentation demonstrates that acemannan is an immunoenhancer and biological response modifier in that it increases lymphocyte response to alloantigen. A proposed mechanism of action involves stimulation of monocytes to release IL-1; in the presence of acemannan, IL-1 has been shown to be released from monocyte cultures. The pharmacologic action of acemannan stimulation of monocytes may explain acemannan activity against viral infection in animals and man.

**Example 4**

**PHARMACOKINETIC BASIS FOR CORRELATION OF IN VITRO AND IN VIVO EFFECTIVENESS OF ACEMANNAN**

To evaluate the pharmacokinetic behavior of acemannan, $^{14}C$-labelled material was given by IP and IV injection and PO administration. Based on the results of previous pilot work, an aqueous dose of 200 mg $^{14}C$-labelled acemannan/200 ml with specific activity of 17.4 cpm/μg was administered to female dogs (approximately 20 mg/kg). Blood, urine and feces samples were taken at appropriate intervals for 48 hours or longer. Organ and tissue samples were taken after sacrifice, and all samples were analyzed for radioactivity using scintillation spectrometry.

Acemannan's kinetic behavior was typical of that seen with most pharmacologic agents; however, its biologic half-life ($t_{1/2}$) was extraordinarily long. Significant absorption occurred by all three routes of administration. Maximum blood levels were achieved after IV injection followed by IP and then PO. Blood levels, which were immediately maximal at 200 μg/ml after IV injection, declined with a $t_{1/2}$ of 50-60 hours; plasma levels were approximately twice those of blood. By comparison, after IP injection blood levels peaked at 45 μg/ml at 24 hours and then declined at a rate similar to that seen with IV; in fact, blood levels were nearly 90% maximal after only 8 hours. With oral administration, blood levels were measurable after 3 hours and peaked at 4-5 μg/ml. Based on the relatively long half-life in blood, a therapeutic dosing interval of approximately 7 days would be justified, considering the time required for three half-lives.

Radiolabeled acemannan distributed mainly in liver and spleen following IP or IV injection. Liver, marrow, thymus, and lymph nodes were primary sites of distribution after oral dosing, a finding consistent with the immunologic sites of action for acemannan. Levels of radiolabeled compound in tissues sampled after 48-52 hours ranged from a low of approximately 1 μg/g brain to a high of 85 μg/g spleen after IV injection. Interestingly, levels in brain and spinal cord were higher (approximately 3 μg/g tissue) after oral, compared to parenteral, administration. This could be the result of the liver's partial breakdown of the polymer into smaller molecular weight fractions during the first pass, thus rendering it capable of penetrating the blood-brain barrier.

In summary, with respect to clinical pharmacokinetic considerations, the data indicate that $^{14}C$-labelled acemannan (1) reaches peak blood levels within 8 hours or less by all routes studied, (2) has a relatively long biologic half-life, which would allow therapeutic dosing intervals of several days, and (3) achieves measurable levels in all tissue systems evaluated, including the central nervous system.

These pharmacokinetic data indicate that acemannan levels in blood and/or tissue can duplicate those levels known after injection or oral administration to produce therapeutic antitumor or antiviral effects in vitro. For example mice implanted with virally-infected Norman Murine Myxosarcoma (NMM) cells and injected IP within 24 hours with 1

mg/kg of acemannan showed 35% survival after 60 days compared to 0% survival in NMM-treated control mice (Peng et al., submitted for publication, 1990). Expected peak blood levels at an IP dose of 1 mg/kg would be on the order of 2 µg/ml (45 µg/ml x 1/20 mg/kg). Acemannan added to cultures of T-lymphocytes at a concentration of only 0.15 µg/ml ($2.6^{10-9}$ M; 60,000 MW) increased the generation of cytotoxic T-cells 230% and increased the functional capacity of generated cytotoxic T-cells by 138% to destroy target cells against which they had been sensitized [Womble et al., Int. J. Immunopharmac. 10(8):967-974 (1988)]. Cytotoxic T-cells are thought to be generated against tumor cells like NMM cells.

Blood levels of 4-5 µg/ml obtained after oral administration of acemannan are also significant, since they correspond to the concentration of acemannan that gives optimal synergism with Zidovudine® (AZT) in vitro. For example, alone 0.001 µg/ml AZT or 3.2 µg/ml acemannan increased the viability of CEM cells infected with $HTLV\text{-}III_{RFII}$ virus by no more than 10%. Together the protective effect of the antiviral combination exceeded 70%. Similarly, a combination of 0.1 µg/ml of AZT and 1 mg/ml acemannan resulted in a protective effect exceeding 80% (Kemp et al. submitted for publication1990).

Thus, in conclusion, this pharmacokinetic study demonstrates that acemannan concentrations at least as great as those known to work in vitro are attainable in vivo.

**Example 5**

**ACEMANNAN TREATMENT OF ALLERGIES RESULTING FROM HYPERSENSITIVITY TO CHEMICALS**

Acemannan was used to ameliorate the inflammatory effect of chemical allergens. Subject T.R., a professional painter, was on the verge of quitting his profession due to wheezing and bronchitis induced by vapors from his paints and solvents. After taking 800 mg/day oral acemannan for five days, his symptoms were relieved. The patient continues to consume 800 mg/day of oral acemannan before work each day and is able to continue painting.

**Example 6**

**ACEMANNAN TREATMENT OF SYMPTOMS ASSOCIATED WITH ASTHMA**

A 76-year-old male, T.T., with a 72-year history of asthma was taking 800 mg acemannan daily for an unrelated condition. After 1 year of therapy, T.T. told the attending physician he had not used his aerosolized bronchodilator for over 1 year and a significant amount was still in the unit. His wife stated that he had averaged 2 units per month for 15 years and had used a hand aerosolizer for 50 years before that for chronic asthma. The patient continues to take 800 mg acemannan daily and no longer has wheezing or chronic bronchitis.

**Example 7**

**ACEMANNAN TREATMENT OF SYMPTOMS ASSOCIATED WITH CYSTIC FIBROSIS**

A college-age female with 6-month history of cystic fibrosis syndrome reported an abrupt return of energy within 2 weeks of instituting oral acemannan therapy at a dose of 800 mg/day.

**SUMMARY**

It is thus believed that the operation and administration of the present invention will be apparent from the foregoing description. While the method and techniques shown and described have been characterized as being preferred, it will be obvious that various changes and modifications may be made therein without departing from the scope of the invention as defined in the following claims.

## Claims

1. The use of an acetylated mannan in the manufacture of a medicament for reducing symptoms associated with chronic respiratory diseases.

2. The use according to claim 1, wherein said acetylated mannan is acemannan.

## Patentansprüche

1. Verwendung eines acetylierten Mannans bei der Herstellung eines Arzneimittels zum Vermindern von Symptomen, die mit chronischen respiratorischen Erkrankungen verbunden sind.

2. Verwendung nach Anspruch 1, wobei das acetylierte Mannan Acemannan ist.

## Revendications

1. Utilisation d'un mannane acétylé pour la fabrication d'un médicament pour la réduction des symptômes associés aux maladies respiratoires chroniques.

2. Utilisation selon la revendication 1, dans laquelle ledit mannane acétylé est l'acémannane.